# EUROPÄISCHE PATENTSCHRIFT

(11) **EP 4 013 356 B1**
(45) Veröffentlichungstag und Bekanntmachung des Hinweises auf die Patenterteilung: **20.03.2024**
(21) Anmeldenummer: 20775816.0
(22) Anmeldetag: 13.08.2020
(51) Int. Cl.: A61F 2/30, A61F 2/46

(54) **OPERATIONS-KIT ZUR ERGÄNZUNG EINES GELENKS**
SURGICAL KIT FOR ADDITION TO A JOINT
KIT CHIRURGICAL DESTINÉ À ÊTRE AJOUTÉ À UNE ARTICULATION

(30) Priorität: 16.08.2019 DE 102019005770
(43) Veröffentlichungstag der Anmeldung: 22.06.2022
(73) Patentinhaber: Erdmann, Alfons, 51429 Bergisch Gladbach (DE); Eyerer, Peter, 76228 Karlsruhe (DE)
(72) Erfinder: Erdmann, Alfons, 51429 Bergisch Gladbach (DE); Eyerer, Peter, 76228 Karlsruhe (DE)
(74) Vertreter: Fischer, Uwe
(86) Internationale Anmeldenummer: PCT/DE2020/200070
(87) Internationale Veröffentlichungsnummer: WO 2021/032257

(56) Entgegenhaltungen:
- WO-A1-2009/129262
- WO-A2-2004/017861
- US-A- 4 313 232
- US-A1- 2007 233 246

## Beschreibung

Die Erfindung liegt auf dem Gebiet der Mechanik, der Werkstoffkunde, der Medizintechnik und des Maschinenbaus und bezieht sich konkret auf eine Zusammenstellung von Bauteilen, die gemeinsam dazu eingerichtet sind, ein Gelenk eines Lebewesens, beispielsweise eines Menschen, zu ergänzen.

Gelenke von Lebewesen, insbesondere solche an Extremitäten, jedoch auch andere Gelenke unterliegen einem dauernden Verschleiß oder können durch Missgestaltungen, Abnutzung oder Unfälle derart beschädigt oder verformt sein, dass ihre Funktion eingeschränkt ist oder dass sie Schmerz verursachen. Beispielsweise kann durch langen Gebrauch oder Fehlstellung der Gelenkknorpel verschlissen oder abgenutzt werden, so dass er langfristig seine Funktion nicht weiter erfüllt.

Für derartige Fälle ist es bekannt, Gelenke im Rahmen von Operationen ganz oder teilweise zu ersetzen. Oft wird dabei eines oder beide der Gelenkelemente, beispielsweise die Gelenkkugel oder eine Gelenkpfanne, oder allgemein eines der beiden Gelenkteile komplett durch eine Endoprothese ersetzt. Ein solcher Ersatz erfordert eine Operation, bei der wenigstens ein Teil des bestehenden Gelenks entfernt und ein neues Gelenkteil mit einem Knochen verbunden wird. WO 2009/126262 A1 offenbart Vorrichtungen und Verfahren zur Immobilisierung von Facettengelenken an der Wirbelsäule.

Der vorliegenden Erfindung liegt die Aufgabe zugrunde, ein Operations-Kit zur Verfügung zu stellen, das eine Ergänzung eines Gelenks mit einem möglichst geringen operativen Eingriff ermöglicht.

Die Erfindung bezieht sich zur Lösung der Aufgabe auf ein Operations-Kit zur Ergänzung eines Gelenks eines Lebewesens, insbesondere eines Menschen, welches zwei gegeneinander bewegbare Gelenkelemente aufweist, zwischen denen ein Gelenkspalt angeordnet ist, mit einem ersten Einsatzteil mit einer ersten Längsachse und zwei von dieser durchsetzten Stirnseiten und wenigstens einer Umfangsfläche, das an wenigstens einer Umfangsfläche wenigstens ein Fixierelement, insbesondere eine Erhebung zur Verankerung in einer Ausnehmung eines ersten Gelenkelementes aufweist, einem zweiten Einsatzteil mit einer zweiten Längsachse und mit zwei von dieser durchsetzten Stirnseiten und wenigstens einer Umfangsfläche, das an wenigstens einer Umfangsfläche wenigstens ein Fixierelement, insbesondere eine Erhebung zur Verankerung in einer Ausnehmung eines zweiten Gelenkelementes aufweist, sowie einer Einlegescheibe zum Einfügen zwischen den Gelenkelementen in den Gelenkspalt, und mit einem Positionierelement zur Positionierung der Einlegescheibe zwischen dem ersten und zweiten Einsatzteil, wobei das erste und/oder zweite Einsatzteil jeweils als zylindrische Hülse ausgebildet sind.

Unter einem Operations-Kit wird hierbei eine Zusammenstellung von Gegenständen oder Bauteilen verstanden, die gemeinsam in ein Gelenk eingefügt werden können und derart aufeinander abgestimmt sind, dass sie gemeinsam das Gelenk ergänzen und seine Funktionsfähigkeit wieder herstellen. Potentiell kann das Operations-Kit außer den im Gelenk langfristig verbleibenden Teilen auch zusätzlich solche Teile umfassen, die nur vorübergehend im Rahmen einer Operation benutzt werden und entweder vorübergehend als Hilfsteile in das Gelenk eingeführt und am Ende der Operation oder nach einem Heilungsprozess entfernt werden oder als Werkzeuge zum Einbringen von Bauteilen in das Gelenk oder zum Manipulieren von Bauteilen im Gelenk dienen.

Die im Gelenk langfristig verbleibenden Teile umfassen ein erstes Einsatzteil, das in ein erstes Gelenkelement eingesetzt wird, sowie ein zweites Einsatzteil, das in ein zweites Gelenkelement eingesetzt wird. Das erste und zweite Einsatzteil sind einander gegenüberliegend auf beiden Seiten des Gelenkspalts positioniert, in den eine Einlegescheibe derart einbringbar ist, dass sie zwischen den beiden Gelenkelementen angeordnet ist und diese voneinander am Gelenkspalt beabstandet. Ein Positionierelement dient dazu, die Einlegescheibe innerhalb des Gelenkspalts im Bereich des ersten und zweiten Einsatzteils zu halten und damit in einem Bereich des Gelenks, in dem die Einlegescheibe einen wesentlichen Teil der Gelenkbelastung, d. h. der Kraft, mit der die beiden Gelenkelemente zusammengedrückt werden, aufnehmen kann. Dabei kann die Einlegescheibe relativ zu einem der beiden Gelenkelemente fixiert oder an einem der beiden Einsatzteile fest oder elastisch fixiert gehalten und relativ zum jeweils anderen Gelenkelement beweglich sein. Sie kann jedoch auch gegenüber beiden Gelenkelementen verschiebbar sein. Das Positionierelement zur Positionierung der Einlegscheibe ist zu diesem Zweck insbesondere mit wenigstens einem der Einsatzteile verbunden.

Die Einlegescheibe kann beispielsweise als runde, Kreisrunde oder ovale Scheibe ausgebildet sein und eine einheitliche Dicke oder auch ein Dickenprofil mit Bereichen unterschiedlicher Dicke aufweisen. Die Dicke kann beispielsweise von der Mitte zum Rand hin stetig oder in Stufen oder auch nur im Randbereich zum Rand hin abnehmen. Die Einlegescheibe kann auch thermisch so vorbehandelt sein, dass eine Spannungsrissbildung nach der Implantation vermieden wird. Hierzu kann die Vorbehandlung so gestaltet sein, dass ohne Belastung in der Einlegescheibe mechanische Spannungen herrschen, die den bei Belastung auftretenden Spannungen entgegenwirken.

Es kann auch vorgesehen sein, dass das Material der Einlegescheibe in den oberflächennahen Bereichen stärker vernetzt ist als in tiefer liegenden Bereichen. Dies kann beispielsweise durch eine selektive Vernetzung durch Bestrahlung oder Wärmebehandlung erreicht sein. Nach einer Bestrahlung kann die Einlegescheibe durch Tempern behandelt werden, wobei die freien Radikale abgebaut werden und ein nachfolgender Molmassenabbau verlangsamt wird.

Die Einlegescheibe kann bei ihrer Herstellung zunächst aus UHMWPE aus einem Halbzeug gefertigt und danach thermisch dreidimensional umgeformt und danach gezielt abgekühlt wird. Die Einlegescheibe kann auch aus pulverförmigen UHMWPE in einer gewünschten Form durch Sintern hergestellt werden. Grundsätzlich kann die Einlegescheibe nach der Herstellung thermisch und/ oder durch Bestrahlung nachbehandelt werden.

Zum Einsetzen der Teile des Operations-Kits, die dauerhaft im Gelenk verbleiben sollen, kann das zu ergänzende Gelenk in eine passende Winkelposition gebracht und von außen fixiert werden. Darauf können entlang einer Bohrachse beide Gelenkelemente wenigstens teilweise durchbohrt werden, wobei eines der Gelenkelemente vollständig durchbohrt wird, um durch dieses hindurch mit dem Bohrer das andere Gelenkelement zu erreichen und in dem anderen Gelenkelement wenigstens eine Sackbohrung zu erzeugen. Durch die Bohrung kann dann zunächst ein Einsatzteil in das vom Operateur aus gesehen distale Gelenkelement durch die Bohrung eingeschoben werden. Danach kann durch dieselbe Bohrung das andere Einsatzteil in das proximale Gelenkelement durch die Bohrung eingebracht werden. Nach dem Einbringen des einen Einsatzteils in das distale Gelenkelement und vor oder nach dem Einbringen des anderen Einsatzteils in das proximale Gelenkelement kann die Einlegescheibe seitlich durch eine minimalinvasive operative Öffnung in den Gelenkspalt eingeschoben werden. Dabei ist die Einlegescheibe bereits mit dem Positionierelement verbunden oder es wird nach dem Einschieben der Einlegscheibe in den Gelenkspalt das Positionierelement mit der Einlegscheibe von der proximalen Seite, d. h. von dem proximalen Gelenkelement, aus verbunden oder gekoppelt. Insbesondere kann, wenn die Einlegscheibe eine durchgehende Öffnung aufweist, von der proximalen Gelenkelementseite her ein strang- oder zapfenförmiges Positionierelement durch die Öffnung der Einlegescheibe hindurchgeschoben werden.

Das erste und das zweite Einsatzteil sind jeweils mit Fixierelementen, insbesondere Erhebungen an ihren Umfangsflächen versehen, die der Verankerung in einer Ausnehmung, insbesondere einer Bohrung, der Gelenkelemente dienen. Mittels der Fixierelemente/Erhebungen werden, bereits während der Operation oder insbesondere durch das auf die Operation folgende heilende Knochenwachstum, die Einsatzteile in den Gelenkelementen befestigt.

In einer Ausgestaltung der Erfindung kann vorgesehen sein, dass das Positionierelement als elastisches strang- oder stabförmiges Element, insbesondere als Schrauben- oder Spiralfeder, als Elastomerzapfen oder als federndes Dämpfungselement, beispielsweise als Tellerfeder oder als Torsionsfeder ausgebildet ist, das an wenigstens einem der beiden Einsatzteile insbesondere an beiden Einsatzteilen, oder an der Einlegescheibe befestigt oder befestigbar ist.

Das Positionierelement kann, ausgehend von einem der Einsatzteile, den Gelenkspalt durchsetzend bis zu den anderen Einsatzteil reichen. Das Positionierelement kann in diesem Fall an einem oder beiden der Einsatzteile befestigt oder befestigbar sein, beispielsweise durch Verkleben, Einklemmen, Verschrauben oder Verschweißen mit einem oder beiden der Einsatzteile. Das Positionierelement kann auch allgemein mit wenigstens einem der Einsatzlteile verbunden, insbesondere einstückigverbunden sein und mit diesem gemeinsam und zusammenhängend hergestellt sein. Insbesondere dann, wenn eines oder beide der Einsatzteile aus einem Metall, beispielsweise Titan, bestehen, kann das stabförmige Element als Schrauben- oder Spiralfeder oder auch als flexibler Draht, insbesondere als flexibler Titandraht ausgebildet sein, ebenfalls aus Metall bestehen und mit einem der Einsatzteile verschweißt oder bereits einstückig mit diesem hergestellt sein. Es kann jedoch auch vorgesehen sein, dass eines oder beide der Einsatzteile als hohle Hülsen ausgebildet sind und jeweils ein Schraubeninnengewinde aufweisen, in das die Wendel eines als Schraubenfeder ausgebildeten Positionierelements einschraubbar ist. Auf diese Weise ist gewährleistet, dass das Positionierelement den Gelenkspalt durchsetzt, in diesem Bereich dauerhaft elastisch verformbar und verschiebbar ist und dabei eine Einlegescheibe halten kann. Dazu kann beispielsweise vorgesehen sein, dass die Einlegescheibe eine durchgehende Öffnung oder einen oder zwei Rohrstutzen aufweist, die oder der zur Aufnahme des Positionierelementes oder zum Einstecken oder Einschrauben in eines der Einsatzteile eingerichtet ist/sind.

Ein Positionierelement kann dann die durchgehende Öffnung der Einlegescheibe durchsetzen oder in einen Rohrstutzen der Einlegescheibe hineinreichen und dort mit der Einlegscheibe verbunden sein. Auch auf diese Weise kann sichergestellt werden, dass die Einlegscheibe flexibel und begrenzt beweglich im Gelenkspalt gehalten ist. Die Verwendung eines flexiblen Positionierelements der genannten Art, insbesondere bei einer Ausführungsform als Schrauben- oder Spiralfeder aus einem Stahl oder einer Titanlegierung, gewährleistet, dass das Positionierelement eine hohe Standzeit und gleichbleibende Bewegbarkeit und Flexibilität über viele Monate und Jahre aufweist.

Wie bereits oben erläutert, kann auch vorgesehen sein, dass das Positionierelement als an der Einlegescheibe befestigter Stutzen, insbesondere Rohrstutzen ausgebildet ist, der mit einem der Einsatzteile verbindbar, insbesondere in eines der beiden Einsatzteile einsteckbar oder einschraubbar ist. Es kann alternativ zur Verbindung mit dem Positionierelement oder zusätzlich dazu auch vorgesehen sein, dass die Einlegescheibe durch eine separate Schraube mit einem Einsatzteil verschraubt ist.

Der Rohrstutzen sollte dabei so kurz ausgebildet sein, dass er die Beweglichkeit der Einlegscheibe im Gelenkspalt nicht behindert. Hierzu kann eine konische Ausnehmung in das Gelenkelement eingebracht werden, das dem Rohrstutzen zugewandt ist. Eine solche Ausnehmung kann während einer Operation durch Fräsen des Gelenkteils eingebracht werden.

Es kann außerdem beispielsweise vorgesehen sein, dass alle Querschnittsflächen des ersten Einsatzteils, die senkrecht auf der ersten Längsachse stehen, kleiner sind als die größte Querschnittsfläche des zweiten Einsatzteils senkrecht zur zweiten Längsachse und dass wenigstens ein Durchmesser der Einlegescheibe, insbesondere bei einer kreisrunden Form der Einlegescheibe der Durchmesser der Einlegscheibe, größer ist als die größte Querschnittsfläche des zweiten Einsatzteils.

Maßgeblich ist dabei, dass das erste Einsatzteil durch die Bohrung, die beide Gelenkelemente durchsetzt, in das erste Gelenkelement eingebracht werden kann. Zu diesem Zweck soll das erste (distale)Einsatzteil durch die Bohrung hindurchgeschoben werden, die das zweite (proximale) Gelenkelement durchsetzt. Zudem muss das zweite Einsatzteil im zweiten Gelenkelement gehalten werden. Aus diesem Grund können vorteilhaft die äußeren Abmaße des ersten Einsatzteils geringer sein als die des zweiten Einsatzteils. Es kann beispielsweise vorgesehen sein, dass die Bohrung im ersten Gelenkelement einen geringeren Durchmesser aufweist als die Bohrung im zweiten Gelenkelement. Hierzu kann zunächst eine Bohrung des geringeren Durchmessers durch beide Gelenkelemente hindurch eingebracht werden und darauf kann eine zweite Bohrung eines größeren Durchmessers nur in das zweite (proximale) Gelenkelement eingebracht werden.

Die Einlegescheibe kann bezüglich ihres Durchmessers so bemessen sein, dass sie deutlich größer ist als die Bohrungen in den Gelenkelementen, so dass sie diese überdeckt und auf den Rändern der Bohrungen an den Gelenkelementen problemlos gleiten kann.

Weiter kann beispielsweise vorgesehen sein, dass eines oder beide Einsatzteile aus einer CoCrMo-Gusslegierung oder einer Titan-Legierung oder generell einer Schmiedelegierung bestehen.

Es kann zudem vorgesehen sein, dass die Einlegscheibe teilweise oder ganz aus einem Elastomer, einem Metall, aus einer Keramik oder insbesondere aus ultrahochmolekularem Polyethylen besteht.

Die genannten Materialien sind in der Endoprothetik seit vielen Jahren bekannt und bewährt.

Das erste und/oder zweite Einsatzteil ist/sind jeweils als zylindrische Hülse ausgebildet.

Dabei kann zudem vorgesehen sein, dass das erste und/oder zweite Einsatzteil jeweils ein Außengewinde aufweist/aufweisen.

Das Außengewinde kann dann die Erhebung am Umfang des/der Einsatzteile bilden. Mittels eines Außengewindes kann eine gute Halterung der Einsatzteile in Bohrungen der Gelenkelemente erreicht werden. Das Einbringen der Einsatzteile ist in einfacher Weise und ohne Zerstörung der Substanz der Gelenkelemente durch Einschrauben möglich. Anstelle eines Außengewindes können auch an den Umfangsflächen umlaufende Stege oder an den Umfangsflächen verteilte Zapfen oder Noppen vorgesehen sein.

In einer oben bereits ansatzweise erläuterten Ausführungsform der Erfindung kann auch vorgesehen sein, dass das erste und/oder zweite Einsatzteil jeweils als hohlzylindrische Hülse ausgebildet sind, wobei insbesondere vorgesehen ist, dass das Positionierteil an dem zweiten Einsatzteil befestigt ist und in das erste Einsatzteil hineinragt, beispielsweise in ein Innengewinde des ersten Einsatzteils einschraubbar ist.

Die Erfindung kann sich zudem auf ein Verfahren zur Ergänzung eines Gelenks eines Wirbeltiers, insbesondere eines Menschen, beziehen, welches zwei gegeneinander bewegbare Gelenkelemente aufweist, zwischen denen ein Gelenkspalt angeordnet ist.

Bei diesem Verfahren kann das oben erläuterte Operationskit verwendet werden. Dabei kann zum Einsetzen der Teile des Operations-Kits, die dauerhaft im Gelenk verbleiben sollen, das zu ergänzende Gelenk in eine passende Winkelposition gebracht und von außen fixiert werden. Darauf können entlang einer Bohrachse beide Gelenkelemente wenigstens teilweise durchbohrt werden, wobei eines der Gelenkelemente vollständig durchbohrt wird, um durch dieses hindurch mit dem Bohrer das andere Gelenkelement zu erreichen und in dem anderen Gelenkelement wenigstens eine Sackbohrung zu erzeugen. Durch die Bohrung kann dann zunächst ein Einsatzteil in das vom Operateur aus gesehen distale Gelenkelement durch die Bohrung eingeschoben werden. Danach kann durch dieselbe Bohrung das andere Einsatzteil in das proximale Gelenkelement durch die Bohrung eingebracht werden. Nach dem Einbringen des einen Einsatzteils in das distale Gelenkelement und vor oder nach dem Einbringen des anderen Einsatzteils in das proximale Gelenkelement kann die Einlegescheibe seitlich durch eine minimalinvasive operative Öffnung in den Gelenkspalt eingeschoben werden. Dabei ist die Einlegescheibe bereits mit dem Positionierelement verbunden oder es wird nach dem Einschieben der Einlegscheibe in den Gelenkspalt das Positionierelement mit der Einlegscheibe von der proximalen Seite, d. h. von dem proximalen Gelenkelement, aus verbunden oder gekoppelt. Insbesondere kann, wenn die Einlegscheibe eine durchgehende Öffnung aufweist, von der proximalen Gelenkelementseite her ein strang- oder zapfenförmiges Positionierelement durch die Öffnung der Einlegescheibe hindurchgeschoben werden.

Die Erfindung kann sich alternativ zu der oben beschriebenen Zusammenstellung von Elementen auch beziehen auf eine Zusammenstellung von Elementen/ein Operations-Kit zur Verbindung zweier jeweils Knochensubstanz enthaltender Körperteile eines Lebewesens,
mit einem ersten Einsatzteil mit einer ersten Längsachse und zwei von dieser durchsetzten Stirnseiten und wenigstens einer Umfangsfläche, das an wenigstens einer Umfangsfläche wenigstens ein Fixierelement, insbesondere eine Erhebung zur Verankerung in einer Ausnehmung eines ersten Gelenkelementes aufweist, einem zweiten Einsatzteil mit einer zweiten Längsachse und mit zwei von dieser durchsetzten Stirnseiten und wenigstens einer Umfangsfläche, das an wenigstens einer Umfangsfläche wenigstens ein Fixierelement, insbesondere eine Erhebung zur Verankerung in einer Ausnehmung eines zweiten Gelenkelementes aufweist, sowie einem mit beiden Einsatzteilen verbindbaren oder verbundenen Positionierelement. Die Erfindung kann sich auch auf ein Verfahren zum Schienen eines gebrochenen Knochens unter Verwendung eines solchen Operationskits beziehen.

Die Einsatzteile können dabei grundsätzlich dieselben Formen annehmen wie weiter oben beschrieben. Das Positionierelement weist bei diesen Ausführungsformen jedoch keine Einlegescheibe auf, sondern ist ausschließlich durch ein Element mit strangförmiger Kontur, beispielsweise einen Elastomerstrang, eine Kunststoff- oder Metallschraube, einen Kunststoff- oder Metallstrang, beispielsweise einen Titanstrang oder einen Titandraht gebildet. Dieses Positionierelement ist im Eingesetzten Zustand im Körper eines Patienten mit beiden Einsatzteilen verbunden. Diese Verbindung kann mit beiden Einsatzteilen gleicher Natur oder unterschiedlicher Natur, das heißt zB mit unterschiedlichen Fügetechniken hergestellt, sein. Beispielsweise kann das Positionierelement mit einem der Einsatzteile bereits vor dem Einsetzen fest verbunden, beispielsweise verklebt, Verlötet, Verschweißt oder einstückig hergestellt sein.

Üblicherweise sieht das Verfahren zur Verwendung eines solchen Operationskits vor, dass bei einem Knochenbruch zunächst in einen ersten der Knochen eine schräge Bohrung eingebracht wird, die in seinem Inneren, beispielsweise in einem Knochenmarkraum endet. Durch diese Bohrung wird ein Bohrwerkzeug eingeführt, das eine Fortsetzung der Bohrung in Axialrichtung des Knochens auf die Bruchstelle zu ermöglicht. Es wird auf diese Weise eine axiale Bohrung in die beiden Bruchstücke des gebrochenen Knochens eingebracht, die die Bruchstelle durchsetzt und sich auf beiden Seiten der Bruchstelle in die Bruchstücke hinein erstreckt. Durch diese Bohrung werden die beiden Einsatzteile in die Knochenbruchstücke eingebracht und in diesen verankert. Dabei ist es vorteilhaft, wenn das Positionierelement in seiner Länge zwischen den Einsätzen so eingestellt wird, dass es mittels der Einsatzteile die beiden Knochenbruchstücke bis zur Berührung mit einer Kompressionskraft zusammenzieht. Zu diesem Zweck kann es vorteilhaft sein, wenn das Positionierelement mit wenigstens einem der Einsatzteile mittels einer axialen Verschraubung verbunden ist, so dass durch Drehen der Verschraubung der Abstand zwischen den beiden Einsatzteilen eingestellt werden kann.

Die Knochenbruchstücke werden auf diese Weise zusammengezogen und relativ zueinander mit einer geringen, einstellbaren Beweglichkeit festgelegt. Damit können die Knochenbruchstücke heilen und zusammenwachsen, ohne dass es eines größeren Eingriffs und der Befestigung einer Schiene an den Bruchstücken bedarf.

Zu dem Operationskit kann außer den Einsatzteilen und dem Positionierelement beispielsweise auch ein Bohrwerkzeug gehören, dass durch eine radiale oder schräg radiale Bohrung in einen Knochen eingeführt werden kann und im Inneren des Knochens die Erzeugung einer axialen Bohrung ermöglicht. Ein solches Bohrwerkzeug umfasst üblicherweise einen Schneidkopf oder Bohrkopf sowie einen Schaft, der flexibel biegbar ist. Ein solcher Schaft kann beispielsweise als relativ steife Schraubenfeder ausgebildet sein, die an ihrem dem Antrieb abgewandten Ende einen Bohrkopf trägt. Zudem kann Ein flexibles Werkzeug zum Einbringen der Einsatzteile und des Positionierelementes in die axiale Bohrung durch die radiale Bohrung hindurch Teil es Operationskits sein. Dieses Werkzeug kann beispielsweise die Gestalt eines Schraubendrehers oder ähnlichen Werkzeugs haben und einen flexiblen Schaft sowie einen Handgriff aufweisen.

Das Operationskit kann beispielsweise auch dadurch ausgestaltet sein, dass
das erste und/oder zweite Einsatzteil jeweils als zylindrische Hülse ausgebildet sind, und
dass das Positionierelement als elastisches strang- oder stabförmiges Element, insbesondere als Schrauben- oder Spiralfeder oder als Elastomerzapfen ausgebildet ist, das in Hohlräume der beiden Hülsen hineinragt und an wenigstens einem der beiden Einsatzteile befestigt oder befestigbar ist und
dass die Einlegscheibe eine durchgehende Öffnung aufweist, die zur Aufnahme des Positionierelementes eingerichtet ist.

Die Erfindung wird im Folgenden anhand von Ausführungsbeispielen in Figuren einer Zeichnung gezeigt und nachfolgend erläutert.

Dabei zeigt:
Figur 1 schematisch zwei Gelenkelemente,
Figuren 2 bis 5 schematisch die beiden Gelenkelemente aus der Figur 1 mit nacheinander eingefügten Teilen des Operations-Kits,
Figuren 6 bis 8 und Figur 11 jeweils schematisch zwei Einsatzteile mit einer zwischen diesen eingefügten Einlegescheibe sowie Positionierelementen,
Figur 9 beispielhaft ein Montagewerkzeug in perspektivischer Darstellung,
Figur 10 ein weiteres Montagewerkzeug mit einem Positionierelement,
Figur 11 eine Längsschnittansicht von zwei Einsatzteilen und einer Einlegescheibe mit einem Werkzeug, sowie
Figuren 12 und 13 die Anwendung von 2 Einsatzteilen und einem Positionierelement bei einem Knochenbruch.

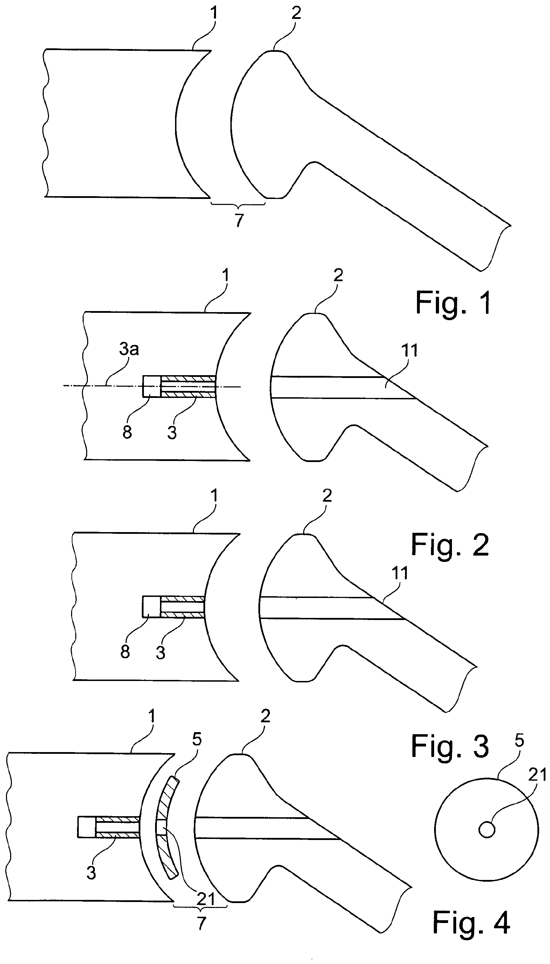

Figur 1 zeigt in einer Seitenansicht ein erstes Gelenkelement 1, das schematisch eine Gelenkpfanne darstellt sowie ein zweites Gelenkelement 2, das schematisch einen Kugelkopf des Gelenks darstellt. Zwischen den beiden Gelenkelementen 1, 2 ist ein Gelenkspalt 7 angeordnet. Im Gelenkspalt befinden sich üblicherweise Knorpelschichten der beiden Gelenkelemente sowie eine Gelenkschmiersubstanz zur Herabsetzung der Reibung bei Bewegung des Gelenks. Die Darstellung des Gelenks ist schematisch und mit der Beschreibung und den Figuren sollen grundsätzlich alle Arten von Gelenken, die beispielsweise im menschlichen Körper vorkommen, wie beispielsweise Scharniergelenke, Drehgelenke oder Gelenke mit mehr als einem oder zwei Freiheitsgraden erfasst werden.

In der Figur 2 ist schematisch in einem Längsschnitt das Gelenk aus der Figur 1 dargestellt, wobei von der Seite des zweiten (proximalen) Gelenkelements 2 aus eine Bohrung 11 in beide Gelenkelemente 1, 2 eingebracht ist. Auf der Seite des ersten (distalen) Gelenkelements 1 ist entlang der Längsachse 3a in die durch die Bohrung geschaffene Ausnehmung 8 ein erstes Einsatzteil 3 in Form einer zylindrischen Hülse aus Titan eingebracht. Das erste Einsatzteil 3 kann beispielsweise in die Ausnehmung 8 eingeschraubt sein. Hierzu kann das erste Einsatzteil 3 ein Außengewinde aufweisen.Das erste Einsatzteil kann aber auch einfach an seiner Außenseite einen oder mehrere Stege aufweisen oder sogar Nuten oder Ausnehmungen, in die im Zuge eines Heilungsprozesses Knochengewebe einwächst, welches das Einsatzteil fixiert.

In einem weiteren Schritt wird, wie in der Figur 3 dargestellt ist, die Bohrung 11 auf der Seite des zweiten Gelenkelements 2 bezüglich ihres Durchmessers erweitert. Das erste Einsatzteil 3 kann auch erst nach der Erweiterung der Bohrung 11 auf Seiten des zweiten Gelenkelements eingebracht werden, da es dann aufgrund seiner Außenmaße einfacher durch das zweite Gelenkelement 2 hindurchbewegbar ist. Grundsätzlich können jedoch die Bohrungen in beiden Gelenkelementen auch denselben Durchmesser aufweisen und behalten, wenn auch die Einsatzteile dieselben Außenmaße aufweisen.

In der Figur 4 ist eine Konfiguration dargestellt, bei der bereits eine Einlegescheibe 5 mit einer durchgehenden Öffnung 21 in den Gelenkspalt 7 eingelegt ist. Die Einlegescheibe 5 wird seitlich vom Rand des Gelenkspalts 7 aus durch eine minimal invasive Öffnung in den Gelenkspalt 7 eingeschoben, so dass die durchgehende Öffnung 21 in etwa mit der Längsachse der Bohrungen in den Gelenkelementen 1, 2 fluchtet. Im rechten Teil der Figur 4 ist die Einlegescheibe 5, die beispielsweise kreisrund oder oval sein kann, in einer Frontalansicht gezeigt.

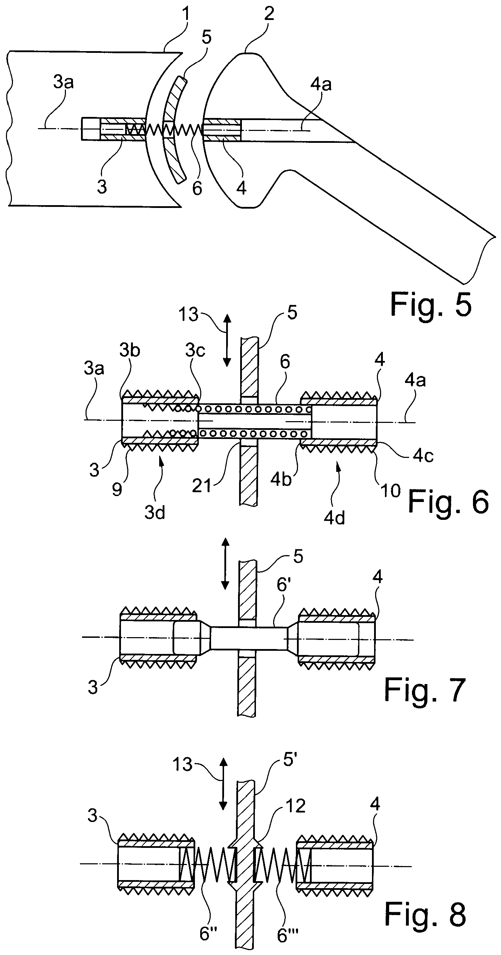

Die Figur 5 zeigt schematisch eine Anordnung mit den beiden Gelenkelementen 1, 2 sowie in diesen eingesetzten Einsatzteilen 3, 4, die beide als metallische, zylindrische bzw. hohlzylindrische Hülsen ausgebildet sind und beispielsweise aus Titan bestehen. Die zwischen den Gelenkelementen 1, 2 und den Einsatzteilen 3, 4 eingeschobene Einlegescheibe 5 besteht vorteilhaft aus hochmolekularem Polyethylen (UHMWPE). Dieses Material ist sehr haltbar und standfest und hat sich in der Endoprothetik seit langen Jahren bewährt.

Mit dem zweiten Einsatzteil 4 ist ein Positionierelement 6 in Form einer Schraubenfeder aus Titan verbunden. Beispielsweise kann die Schraubenfeder 6 an dem zweiten Einsatzteil 4 befestigt, beispielsweise festgeschweißt, sein oder in das zweite Einsatzteil 4 eingeschraubt sein. Zu diesem Zweck kann das zweite Einsatzteil 4 beispielsweise ein Innengewinde aufweisen, in das die Wendel der Schraubenfeder einfädelbar ist.

Das Positionierelement 6 ragt durch die durchgehende Öffnung 21 in der Einlegescheibe 5 hindurch bis zu dem gegenüberliegenden, distalen Einsatzteil 3. Das Einsatzteil 3 weist eine zylindrische Öffnung auf, in die das Positionierelement 6 hineinragt und in der das Positionierelement gehalten wird. Zusätzlich kann das Positionierelement 6 beispielsweise durch Einschrauben in ein Innengewinde des Einsatzteils 3 fixiert werden.

Durch die flexible und elastische Verformbarkeit der Schraubenfeder / des Positionierelements 6 wird die Einlegescheibe 5 im Gelenkspalt 7 flexibel und begrenzt beweglich gehalten. Dadurch kann das Gelenk in allen notwendigen Richtungen bewegt werden, wobei die Einlegscheibe 5 die Reibung der Gelenkelemente aneinander verringert und den auftretenden Abrieb minimiert. Nach jeder Bewegung wird die Einlegscheibe 5 durch das Positionierelement 6 neu in einer neutralen Position zentriert.

Der Außendurchmesser des Positionierelements 6 kann mit dem Durchmesser der Öffnung 21 in der Einlegscheibe 5 übereinstimmen oder geringer sein als der Durchmesser der Öffnung.

In den Figuren 6, 7, 8 und 11 sind der Übersichtlichkeit halber die Gelenkelemente 1, 2 weggelassen und es sind lediglich Elemente des Operations-Kits dargestellt. In der Figur 6 ist in einem Längsschnitt das erste Einsatzteil 3 mit seinen Stirnseiten 3b, 3c und der zylindrischen Umfangsfläche 3d dargestellt. Die Symmetrieachse/Längsachse des ersten Einsatzteils 3 ist mit 3a bezeichnet. Dem ersten Einsatzteil 3 liegt das zweite Einsatzteil 4 gegenüber, das ebenfalls als zylindrische Hülse mit zwei Stirnseiten 4b, 4c und einer Umfangsfläche 4d ausgebildet ist. Die Längsachse des zweiten Einsatzteils 4 ist mit 4a bezeichnet. Die beiden Einsatzteile 3, 4 weisen jeweils Außengewinde 9, 10 auf. Zwischen den Einsatzteilen 3, 4 ist ein Positionierelement 6 in Form einer Schraubenfeder dargestellt, die eine Öffnung 21 in der Einlegescheibe 5 durchsetzt und in die beiden Hohlräume der Hülsen 3, 4 hineinragt. Die Einlegescheibe 5 ist entlang dem Doppelpfeil 13 begrenzt verschiebbar.

In der Figur 7 ist eine Ausführungsform mit zwei Einsatzteilen 3, 4 dargestellt, die als hohlzylindrische Hülsen ausgebildet sind, wobei das Positionierelement 6' als Elastomerstab ausgebildet ist, der eine Keulenform aufweist mit zwei verdickten Enden, die jeweils in die Hohlräume der Hülsen 3, 4 hineinragen und dort gehalten sind. Dabei kann ein Ende des Stabs 6' in dem zweiten Einsatzteil 4 fest fixiert, beispielsweise eingeschraubt sein, während das gegenüberliegende Ende des Stabs 6' in dem ersten Einsatzteil 3 zumindest in Längsrichtung, d. h. entlang der Achse 3a, verschiebbar ist. Damit ist eine Beweglichkeit des Positionierelements 6' gegeben, die eine Verschiebbarkeit der Einlegescheibe 5 gewährleistet.

In der Figur 8 ist eine weitere Ausführungsform dargestellt, bei der die Einsatzteile 3, 4 als hohle metallische Hülsen ausgeführt sind, wobei das Positionierelement in zwei Schraubenfedern 6" und 6‴ geteilt ist. Jede der Schraubenfedern 6", 6‴ ist in einem der Einsatzteile 3, 4 oder an einem der Einsatzteile fixiert und ragt aus diesen heraus in den Gelenkspalt 7 hinein. Die Schraubenfedern 6", 6‴ ragen dabei in jeweils einen Rohrstutzen 12 hinein, der auf je einer Seite der Einlegscheibe 5' angeordnet ist. Die Rohrstutzen sind kurz ausgebildet und mit einem Außenkonus versehen, so dass sie sich an den Gelenkelementen 1, 2 nicht verhaken. Es können anstelle der Rohrstutzen 12 auch einfach Sackbohrungen auf beiden Seiten der Einlegescheibe 5' zur Aufnahme der Enden der Schraubenfedern 6", 6"' vorgesehen sein. Die Schraubenfedern 6", 6‴ sind mit einem Druck in Längsrichtung vorbelastet, so dass sie die Einlegscheibe 5' im Gelenkspalt halten und fixieren. Dennoch ist die Einlegescheibe 5' in Richtung des Doppelpfeils 13 begrenzt beweglich. Durch die Wirkung der Schraubenfedern 6", 6‴ wird die Einlegescheibe 5` nach jeder Bewegung in einer neutralen Position neu zentriert.

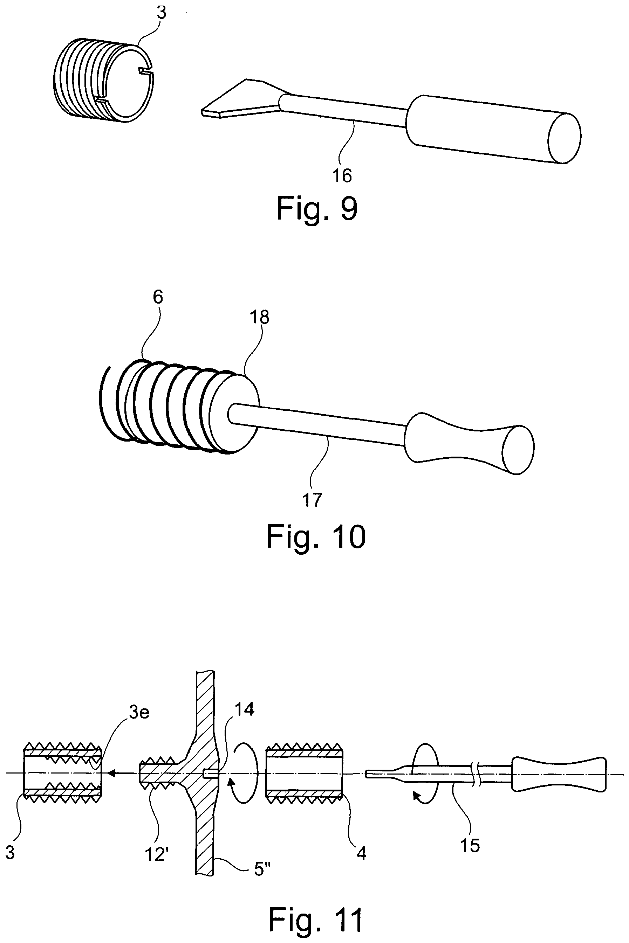

Die Figur 11 zeigt die beiden Einsatzteile 3, 4, mit einem anderen Positionierelement 5 ", wobei das Einsatzteil 3 ein Innengewinde 3e aufweist.

Im Gelenkspalt zwischen den Einsatzteilen 3, 4 ist eine Einlegescheibe 5" angeordnet, die mit einem stutzenförmigen Ansatz 12' verbunden ist, der sich entlang der Längsachse 3a des ersten Einsatzteils 3 erstreckt und in das Innengewinde 3e des ersten Einsatzteils 3 mittels eines Außengewindes einschraubbar ist. Auf der dem stabförmigen Ansatz gegenüberliegenden Seite weist die Einlegescheibe 5" eine Ausnehmung 14, beispielsweise einen Schlitz oder Kreuzschlitz, auf, in die ein schraubenzieherartiges Werkzeug 15 durch die Bohrung 11 im zweiten Gelenkteil und den zylindrischen Hohlraum des zweiten Einsatzteils 4 hindurch formschlüssig eingreifen kann, um die Einlegscheibe 5" im Innengewinde 3e des ersten Einsatzteils 3 zu verschrauben. Das Positionierelement ist in diesem Fall durch den stutzenförmigen Ansatz 12' verwirklicht.

Die Figur 9 zeigt schematisch ein Werkzeug nach Art eines Schraubenziehers 16, der durch die Bohrung 11 hindurch beispielsweise zum Festschrauben des ersten Einsatzteils 3 mittels eines in diesem an seiner Stirnseite vorgesehenen Schlitzes erlaubt.

In der Figur 10 ist ein Werkzeug 17 dargestellt, das an seinem Ende einen Zylinder 18 aufweist, an dessen Umfangsfläche ein Positionierelement 6 in Form einer Schraubenfeder gehalten ist. Durch Drehung des Werkzeugs 17 um seine Längsachse kann die Schraubenfeder 6 beispielsweise in eines der Einsatzteile 3, 4 eingedreht und dort beispielsweise in einem Innengewinde fixiert werden.

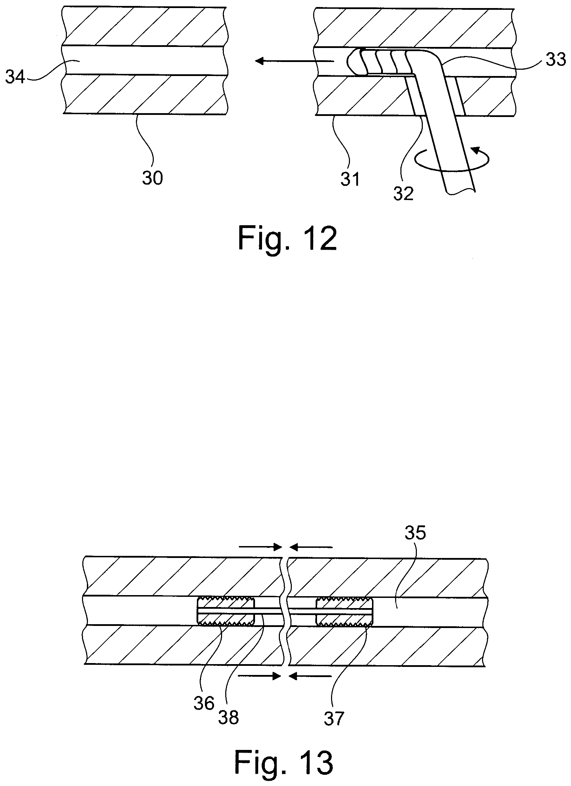

In der Figur 12 sind zwei Knochenbruchstücke 30, 31 gezeigt, die miteinander zur Heilung verbunden werden sollen. In eines der Knochenbruchstücke ist eine radiale Bohrung 32 eingebracht, durch die ein Bohrwerkzeug 33 in den Markkanal des Knochens eingeführt werden kann. Das Bohrwerkzeug 33 ist flexibel und kann mittels einer biegsamen Welle angetrieben werden. Mit dem Bohrwerkzeug kann ein definierter Kanal35 in das Knocheninnere der beiden Bruchstücke 30, 31 eingebracht werden, insbesondere kann der Markkanal 34 in beiden Bruchstücken erweitert werden.

Nach der Erzeugung des definierten Kanals 35 können in diesem die beiden Einsatzteile 36 und 37 beiderseits der Bruchstelle in die beiden Knochenbruchstücke 30, 31 eingebracht und dort fixiert werden, wie in Figur 13 dargestellt ist. Die beiden Einsatzteile 36, 37 können mittels eines Positionierelements 38, das grundsätzlich ebenso gestaltet sein kann, wie die oben im Zusammenhang mit der Gelenkendoprothese beschriebenen Positionierelemente, miteinander verbunden werden. Dabei können die Knochenbruchstücke mit einer Kompressionskraft durch das Positionierelement zusammengepresst werden. Beispielsweise können die beiden Einsatzteile jeweils Innengewinde mit verschiedenem Drehsinn aufweisen, in die das Positionierelement, das mit entsprechend passenden Gegengewinden ausgestattet ist, eingeschraubt werden kann. Beim Einschrauben des Positionierelementes können auf diese Weise die beiden Einsatzteile aufeinander zu bewegt werden, um eine Kompressionskraft auf die Knochenbruchstücke zu erzeugen.

Das Positionierelement kann dennoch eine gewisse Elastizität aufweisen, um die Bruchgefahr zu reduzieren. Die resultierende, geringe Bewegbarkeit beeinträchtigt die Heilung der Knochenbruchstücke nicht.

Durch die Erfindung wird es möglich, ein nicht in wünschenswerter Weise funktionierendes Gelenk durch eine Einlegescheibe zu ergänzen, die im Gelenkspalt die Reibungs- und Bewegungsverhältnisse der Gelenkelemente gegeneinander verbessert. Die Einlegescheibe wird dabei möglichst in beweglicher Form gehalten, jedoch derart, dass sie regelmäßig im Gelenk neu positioniert und zentriert wird. Damit wird eine Ergänzung eines Gelenks zur Verbesserung seiner Funktionsfähigkeit mit minimal invasiven Mitteln ermöglicht.

### Bezugszeichenliste

- 1: Gelenkelement
- 2: Gelenkelement
- 3: Einsatzteil, Hülse
- 3a: Längsachse, Symmetrieachse
- 3b: Stirnseite
- 3c: Stirnseite
- 3d: Umfangsfläche
- 3e: Innengewinde
- 4: Einsatzteil, Hülse
- 4a: Längsachse
- 4b: Stirnseite
- 4c: Stirnseite
- 4d: Umfangsseite
- 5: Einlegescheibe
- 5': Einlegescheibe
- 5": Einlegescheibe
- 6: Positionierelement
- 6`: Positionierelement, Stab
- 6": Schraubenfeder
- 6‴: Schraubenfeder
- 7: Gelenkspalt
- 8: Ausnehmung
- 9: Außengewinde
- 10: Außengewinde
- 11: Bohrung
- 12: Rohrstutzen
- 12': Ansatz
- 13: Doppelpfeil
- 14: Ausnehmung
- 15: Werkzeug
- 16: Werkzeug
- 17: Werkzeug
- 18: Zylinder
- 21: Öffnung in 5

## Patentansprüche

1. Operations-Kit zur Ergänzung eines Gelenks eines Wirbeltiers, insbesondere eines Menschen, welches zwei gegeneinander bewegbare Gelenkelemente (1, 2) aufweist, zwischen denen ein Gelenkspalt (7) angeordnet ist, mit einem ersten Einsatzteil (3) mit einer ersten Längsachse (3a) und zwei von dieser durchsetzten Stirnseiten(3b, 3c) und wenigstens einer Umfangsfläche (3d), das an wenigstens einer Umfangsfläche wenigstens ein Fixierelement, insbesondere eine Erhebung (9) zur Verankerung in einer Ausnehmung (8) eines ersten Gelenkelementes (1) aufweist,
einem zweiten Einsatzteil (4) mit einer zweiten Längsachse (4a) und mit zwei von dieser durchsetzten Stirnseiten (4b, 4c) und wenigstens einer Umfangsfläche (4d), das an wenigstens einer Umfangsfläche wenigstens ein Fixierelement, insbesondere eine Erhebung (10) zur Verankerung in einer Ausnehmung (11) eines zweiten Gelenkelementes (2) aufweist, sowie
einer Einlegescheibe (5, 5', 5") zum Einfügen zwischen den Gelenkelementen (1, 2) in den Gelenkspalt (7),
und mit einem Positionierelement (6, 6', 6", 6‴) zur Positionierung der Einlegescheibe (5, 5') zwischen dem ersten und zweiten Einsatzteil, **dadurch gekennzeichnet, dass** das erste und/oder zweite Einsatzteil (3, 4) jeweils als zylindrische Hülse ausgebildet sind.

2. Operations-Kit nach Anspruch 1, wobei das Positionierelement (6, 6', 6", 6‴) als elastisches strang- oder stabförmiges Element, insbesondere als Schrauben- oder Spiralfeder, Tellerfeder oder Torsionsfeder oder als Draht oder als Elastomerzapfen ausgebildet ist, das an wenigstens einem der beiden Einsatzteile (3, 4), insbesondere an beiden Einsatzteilen, oder an der Einlegescheibe befestigt oder befestigbar ist oder mit diesem, insbesondere bei gleicher Materialwahl, einstückig hergestellt ist.

3. Operations-Kit nach Anspruch 1 oder 2, wobei die Einlegescheibe (5) eine durchgehende Öffnung (11) oder einen oder zwei Rohrstutzen (12, 12') aufweist, die oder der zur Aufnahme des Positionierelementes (6, 6', 6", 6‴) oder zum Einstecken oder Einschrauben in eines der Einsatzteile (3, 4) eingerichtet ist/sind.

4. Operations-Kit nach Anspruch 1, 2 oder 3, wobei das Positionierelement als an der Einlegscheibe (5', 5") befestigter Stutzen, insbesondere Rohrstutzen (12, 12') ausgebildet ist, der mit einem der Einsatzteile 3, 4) verbindbar, insbesondere in eines der beiden Einsatzteile einsteckbar oder einschraubbar und insbesondere durch eine separate Schraube mit einem Einsatzteil verschraubbar ist.

5. Operations-Kit nach Anspruch 1, wobei alle Querschnittsflächen des ersten Einsatzteils (3), die senkrecht auf der ersten Längsachse (3a) stehen, kleiner oder gleich so groß sind als/wie die größte Querschnittsfläche des zweiten Einsatzteils (4) senkrecht zur zweiten Längsachse (4a) wobei insbesondere wenigstens ein Durchmesser der Einlegscheibe (5, 5', 5"), insbesondere bei einer kreisrunden Form der Einlegscheibe der Durchmesser der Einlegscheibe, größer ist als die größte Querschnittsfläche des zweiten Einsatzteils (4).

6. Operations-Kit nach Anspruch 1 oder einem der folgenden, wobei eines oder beide Einsatzteile (3, 4) aus einer CoCrMo-Gusslegierung oder einer Titan-Legierung oder einer Schmiedelegierung bestehen.

7. Operations-Kit nach Anspruch 1 oder einem der folgenden, wobei die Einlegscheibe (5, 5', 5") teilweise oder ganz aus einem Elastomer, einem Metall, aus einer Keramik oder insbesondere aus ultrahochmolekularem Polyethylen, UHMWPE, besteht.

8. Operations-Kit nach Anspruch 1 oder einem der folgenden, wobei das erste und/oder zweite Einsatzteil jeweils ein Außengewinde (9, 10) aufweisen.

9. Operations-Kit nach Anspruch 1 oder einem der folgenden, wobei das erste und zweite Einsatzteil (3, 4) jeweils als hohlzylindrische Hülse ausgebildet sind, wobei insbesondere vorgesehen ist, dass das Positionierteil (6, 6') an dem zweiten Einsatzteil (4) befestigt ist und in das erste Einsatzteil hineinragt, insbesondere in ein Innengewinde (3e) des ersten Einsatzteils (3) einschraubbar ist.

10. Operations-Kit nach Anspruch 1 oder einem der folgenden, wobei das erste und zweite Einsatzteil (3, 4) jeweils als zylindrische Hülse ausgebildet sind, und dass das Positionierelement (6, 6', 6", 6‴) als elastisches strang- oder stabförmiges Element, insbesondere als Schrauben- oder Spiralfeder oder als Elastomerzapfen ausgebildet ist, das in Hohlräume der beiden Hülsen hineinragt und an wenigstens einem der beiden Einsatzteile (3, 4) befestigt oder befestigbar ist und dass die Einlegscheibe (5) eine durchgehende Öffnung (11) aufweist, die zur Aufnahme des Positionierelementes (6, 6', 6", 6‴) eingerichtet ist.

## Claims

1. Surgical kit for adding to a joint of a vertebrate, in particular of a human, which surgical kit has two joint elements (1, 2) which are movable relative to each other and between which there is a joint space (7), said surgical kit having a first insert part (3) with a first longitudinal axis (3a) and two end faces (3b, 3c) through which said longitudinal axis passes, and at least one circumferential face (3d), said first insert part (3) having, on at least one circumferential face, at least one fixing element, in particular an elevation (9) for anchoring in a recess (8) in a first joint element (1); a second insert part (4) with a second longitudinal axis (4a) and with two end faces (4b, 4c) through which said longitudinal axis passes, and at least one circumferential face (4d), said second insert part (4) having, on at least one circumferential face, at least one fixing element, in particular an elevation (10) for anchoring in a recess (11) in a second joint element (2); and an insertion plate (5, 5', 5'') for introducing into the joint space (7) between the joint elements (1, 2); and a positioning element (6, 6', 6", 6‴) for positioning the insertion plate (5, 5') between the first and the second insert parts, **characterized in that** the first and/or the second insert part (3, 4) are each designed as a cylindrical sleeve.

2. Surgical kit according to Claim 1, wherein the positioning element (6, 6', 6", 6‴) is designed as an elastic strand-shaped or rod-shaped element, in particular as a helical or spiral spring, disc spring or torsion spring or as a wire or as an elastomer pin, which is fastened or fastenable to at least one of the two insert parts (3, 4), in particular to both insert parts, or to the insertion plate or is made in one piece therewith, in particular with the same choice of material.

3. Surgical kit according to Claim 1 or 2, wherein the insert plate (5) has a through-opening (11) or one or two pipe sockets (12, 12'), which through-opening or pipe sockets are designed to receive the positioning element (6, 6', 6'', 6‴) or to be plugged or screwed into one of the insert parts (3, 4).

4. Surgical kit according to Claim 1, 2 or 3, wherein the positioning element is designed as a socket, in particular a pipe socket (12, 12'), which is fastened to the insertion plate (5', 5") and which is connectable to one of the insert parts (3, 4), in particular pluggable or screwable into one of the two insert parts, and in particular screwable to an insert part by a separate screw.

5. Surgical kit according to Claim 1, wherein all the cross-sectional areas of the first insert part (3), which are perpendicular to the first longitudinal axis (3a), are less than or equal to the largest cross-sectional area of the second insert part (4) perpendicular to the second longitudinal axis (4a), wherein in particular at least one diameter of the insertion plate (5, 5', 5''), in particular the diameter of the insertion plate in the case of a circular shape of the insertion plate, is greater than the largest cross-sectional area of the second insert part (4).

6. Surgical kit according to Claim 1 or one of the subsequent claims, wherein one or both insert parts (3, 4) are made of a CoCrMo cast alloy or a titanium alloy or a forged alloy.

7. Surgical kit according to Claim 1 or one of the subsequent claims, wherein the insertion plate (5, 5', 5'') is made partially or entirely from an elastomer, a metal, a ceramic or, in particular, ultra-high molecular weight polyethylene (UHMWPE).

8. Surgical kit according to Claim 1 or one of the subsequent claims, wherein the first and/or the second insert part each have an external thread (9, 10).

9. Surgical kit according to Claim 1 or one of the subsequent claims, wherein the first and the second insert part (3, 4) are each designed as a hollow cylindrical sleeve, wherein it is provided in particular that the positioning part (6, 6') is fastened to the second insert part (4) and protrudes into the first insert part, in particular is screwable into an internal thread (3e) of the first insert part (3).

10. Surgical kit according to Claim 1 or one of the subsequent claims, wherein the first and the second insert part (3, 4) are each designed as a cylindrical sleeve, and the positioning element (6, 6', 6", 6‴) is designed as an elastic strand-shaped or rod-shaped element, in particular as a helical or spiral spring or as an elastomer pin, which protrudes into cavities of the two sleeves and is fastened or fastenable to at least one of the two insert parts (3, 4), and the insertion plate (5) has a through-opening (11) which is designed to receive the positioning element (6, 6', 6", 6‴).

## Revendications

1. Kit chirurgical destiné à être ajouté à une articulation d'un vertébré, notamment d'un être humain, qui présente deux éléments d'articulation (1, 2) mobiles l'un par rapport à l'autre, entre lesquels est agencé un interstice d'articulation (7), avec une première pièce d'insertion (3) avec un premier axe longitudinal (3a) et deux côtés frontaux (3b, 3c) traversés par celui-ci et au moins une surface périphérique (3d), qui présente sur au moins une surface périphérique au moins un élément de fixation, notamment un bossage (9) pour l'ancrage dans un évidement (8) d'un premier élément d'articulation (1), une deuxième pièce d'insertion (4) avec un deuxième axe longitudinal (4a) et avec deux côtés frontaux (4b, 4c) traversés par celui-ci et au moins une surface périphérique (4d), qui présente sur au moins une surface périphérique au moins un élément de fixation, notamment un bossage (10) pour l'ancrage dans un évidement (11) d'un deuxième élément d'articulation (2), ainsi qu'une rondelle intercalaire (5, 5', 5") destinée à être interposée entre les éléments d'articulation (1, 2) dans l'interstice d'articulation (7),
et avec un élément de positionnement (6, 6', 6", 6‴) pour le positionnement de la rondelle intercalaire (5, 5') entre la première et la deuxième pièce d'insertion, **caractérisé en ce que** la première et/ou la deuxième pièce d'insertion (3, 4) sont chacune réalisées sous forme de manchon cylindrique.

2. Kit chirurgical selon la revendication 1, dans lequel l'élément de positionnement (6, 6', 6", 6''') est réalisé sous forme d'élément élastique en forme de barre ou de tige, notamment sous forme de ressort hélicoïdal ou spiral, de rondelle-ressort ou de ressort de torsion, ou sous forme de fil métallique ou sous forme de pivot en élastomère, qui est fixé ou peut être fixé sur au moins l'une des deux pièces d'insertion (3, 4), notamment sur les deux pièces d'insertion, ou sur la rondelle intercalaire, ou qui est fabriqué d'un seul tenant avec celle-ci, notamment lorsque le matériau choisi est identique.

3. Kit chirurgical selon la revendication 1 ou 2, dans lequel la rondelle intercalaire (5) présente une ouverture traversante (11) ou un ou deux embouts tubulaires (12, 12') adaptés pour recevoir l'élément de positionnement (6, 6', 6'', 6''') ou pour être enfichés ou vissés dans l'une des pièces d'insertion (3, 4).

4. Kit chirurgical selon la revendication 1, 2 ou 3, dans lequel l'élément de positionnement est réalisé sous forme d'embout, notamment d'embout tubulaire (12, 12'), fixé à la rondelle intercalaire (5', 5"), qui peut être relié à l'une des pièces d'insertion 3, 4), notamment qui peut être enfiché ou vissé dans l'une des deux pièces d'insertion et notamment qui peut être vissé à une pièce d'insertion par une vis séparée.

5. Kit chirurgical selon la revendication 1, dans lequel toutes les surfaces de section transversale de la première pièce d'insertion (3) qui sont perpendiculaires au premier axe longitudinal (3a) sont inférieures ou égales à la plus grande surface de section transversale de la deuxième pièce d'insertion (4) perpendiculaire au deuxième axe longitudinal (4a), notamment au moins un diamètre de la rondelle intercalaire (5, 5', 5''), notamment dans le cas d'une forme circulaire de la rondelle intercalaire le diamètre de la rondelle intercalaire, étant supérieur à la plus grande surface de section transversale de la deuxième pièce d'insertion (4) .

6. Kit chirurgical selon la revendication 1 ou l'une quelconque des suivantes, dans lequel l'une ou les deux pièces d'insertion (3, 4) sont constituées d'un alliage de fonderie CoCrMo ou d'un alliage de titane ou d'un alliage de forge.

7. Kit chirurgical selon la revendication 1 ou l'une quelconque des suivantes, dans lequel la rondelle intercalaire (5, 5', 5'') est constituée partiellement ou totalement d'un élastomère, d'un métal, d'une céramique ou notamment de polyéthylène de poids moléculaire ultra élevé, UHMWPE.

8. Kit chirurgical selon la revendication 1 ou l'une quelconque des suivantes, dans lequel la première et/ou la deuxième pièce d'insertion présentent chacune un filetage extérieur (9, 10).

9. Kit chirurgical selon la revendication 1 ou l'une quelconque des suivantes, dans lequel la première et la deuxième pièce d'insertion (3, 4) sont chacune réalisées sous forme de manchon cylindrique creux, il étant notamment prévu que la pièce de positionnement (6, 6') est fixée à la deuxième pièce d'insertion (4) et pénètre dans la première pièce d'insertion, notamment en pouvant être vissée dans un filetage intérieur (3e) de la première pièce d'insertion (3).

10. Kit chirurgical selon la revendication 1 ou l'une quelconque des suivantes, dans lequel la première et la deuxième pièce d'insertion (3, 4) sont chacune réalisées sous forme de manchon cylindrique, et l'élément de positionnement (6, 6', 6'', 6''') est réalisé sous forme d'élément élastique en forme de barre ou de tige, notamment sous forme de ressort hélicoïdal ou spiral ou sous forme de pivot en élastomère, qui pénètre dans des cavités des deux manchons et qui est fixé ou peut être fixé à au moins l'une des deux pièces d'insertion (3, 4) et
la rondelle intercalaire (5) présente une ouverture traversante (11) qui est adaptée pour recevoir l'élément de positionnement (6, 6', 6", 6‴).
